# EUROPÄISCHE PATENTANMELDUNG

(11) **EP 0 671 426 A1**
(43) Veröffentlichungstag der Anmeldung: **13.09.1995**
(21) Anmeldenummer: 95101726.8
(22) Anmeldetag: 09.02.1995
(51) Int. Cl.: C08G 18/78, C08G 18/79, C08G 18/73

(54) **Modifizierte (cyclo)aliphatische Polyisocyanatmischungen, Verfahren zu ihrer Herstellung und ihre Verwendung**

(30) Priorität: 17.02.1994 DE 4405054
(71) Anmelder: BASF AKTIENGESELLSCHAFT, D-67056 Ludwigshafen (DE)
(72) Erfinder: Wolff, Stefan, Dr., D-67117 Limburgerhof (DE); Brandt, Peter, Dr., D-67059 Ludwigshafen (DE); Bruchmann, Bernd, Dr., D-67069 Ludwigshafen (DE); Laqua, Gerhard, Dr., D-68167 Mannheim (DE); Siebenhaar, Ursula, Dr., B-02920 Kalluthout (BE); Tesch, Helmut, Dr., D-67127 Rödersheim-Gronau (DE); Renz, Hans, Dr., D-67149 Meckenheim (DE); Witzel, Tom, Dr., D-67069 Ludwigshafen (DE); Merger, Franz, Dr., D-67227 Frankenthal (DE)

(57) **Zusammenfassung**

Gegenstände der Erfindung sind mit Urethan-, Allophanat-, Harnstoff-, Biuret-, Carbodiimid-, Uretdion- und/oder Isocyanuratgruppen modifizierte Polyisocyanatmischungen mit einem Isocyanatgehalt von 1 bis 34 Gew.-%, die erhältlich sind aus Diisocyanaten mit Isocyanatgruppen unterschiedlicher Reaktivität aus der Gruppe 2-Butyl-2-ethyl-pentan-diisocyanat-1,5, 2-(3-Isocyanatopropyl)-cyclohexylisocyanat-1 und/oder 1-(Isocyanatomethyl)-1-(3-isocyanatopropyl)-cyclohexan, Verfahren zu ihrer Herstellung und ihre Verwendung.

## Beschreibung

Erfindungsgegenstände sind mit Urethan-, Allophanat-, Harnstoff-, Biuret-, Carbodiimid-, Uretdion- und/oder Isocyanuratgruppen modifizierte Polyisocyanatzusammensetzungen mit einem Isocyanatgehalt von 1 bis 34 Gew.-%, bezogen auf das Gesamtgewicht, die hergestellt werden aus Diisocyanaten mit Isocyanatgruppen unterschiedlicher Reaktivität, ausgewählt aus der Gruppe 2-Butyl-2-ethyl-pentan-diisocyanat-1,5, 2-(3-Isocyanatopropyl)-cyclohexylisocyanat-1 und/oder 1-(Isocyanatomethyl)-1-(3-isocyanatopropyl)-cyclohexan, Verfahren zu ihrer Herstellung und ihre Verwendung.

Polyisocyanat-Polyadditionsprodukte können bekanntermaßen hergestellt werden durch Umsetzung von organischen Polyisocyanaten mit Verbindungen, die mit NCO-Gruppen reaktive Wasserstoffatome, wie Wasserstoffatome von z.B. Hydroxyl-, Amino-, Mercapto- oder Carboxylgruppen, gebunden haben, in Gegenwart oder Abwesenheit von Katalysatoren, Lösungsmitteln und anderen Zusatzstoffen. Häufig ist es zweckmäßig oder sogar notwendig, die organischen Polyisocyanate vor ihrer Verwendung zur Bildung von Polyisocyanat-Polyadditionsprodukten für spezielle Anwendungen zu modifizieren. Gründe hierfür können beispielsweise sein: Die Erniedrigung des Dampfdrucks, eine Änderung der Viskosität und damit ihrer Verarbeitbarkeit, der Funktionalität und der Reaktivität sowie insbesondere die Erzielung spezieller mechanischer Eigenschaften bei den Endprodukten. Die Modifizierung erfolgt durch eine partielle chemische Veränderung der Isocyanatgruppen, so daß höhermolekulare Polyisocyanatzusammensetzungen mit noch freien, reaktiven Isocyanatgruppen resultieren. Als technisch wichtigste Modifizierungsreaktionen sind z.B. die Di- und Trimerisierung zu Uretdion- und Isocyanuratgruppen und die Bildung von Carbodiimid-, Biuret-, Harnstoff-, Urethan- und/oder Allophanatgruppen zu nennen. Durch geeignete Katalysatoren und Reaktionsbedingungen ist es im allgemeinen möglich eine selektive Modifizierung zu erzielen.

Zur Herstellung derartiger Urethan-, Allophanat-, Harnstoff-, Biuret-, Carbodiimid-, Uretdion- und/oder Isocyanuratgruppen enthaltender Polyisocyanatmischungen können z.B. aliphatische oder cycloaliphatische Diisocyanate oder Mischungen aus aliphatischen und cycloaliphatischen Diisocyanaten in Gegenwart eines geeigneten Katalysators lösungsmittelfrei oder unter Zusatz eines Lösungsmittels und gegebenenfalls eines Zusatzstoffes, zweckmäßigerweise bei erhöhten Temperaturen, beispielsweise bei 60 bis 140°C, bis zum Erreichen des gewünschten Umsatzes zur Reaktion gebracht werden. Anschließend können die Katalysatoren thermisch und/oder durch Zusatz eines Katalysatorgifts desaktiviert und gegebenenfalls die überschüssigen, monomeren (cyclo)aliphatischen Diisocyanate und die gegebenenfalls zugesetzten Lösungsmittel abgetrennt werden. Vorzugsweise erfolgt die Abtrennung durch Destillation unter vermindertem Druck mit Hilfe eines Dünnschichtverdampfers. Nach solchen Verfahren hergestellte modifizierte Polyisocyanatmischungen können mit höhermolekularen und/oder niedermolekularen Polyhydroxylverbindungen, z.B. Polyether-polyolen, Polyester-polyolen oder Polyhydroxyacrylaten, zu NCO-Gruppen enthaltenden Prepolymeren umgesetzt werden. Ferner können die Isocyanatgruppen der modifzierten Polyisocyanatzusammensetzungen oder NCO-Prepolymeren verkappt oder blockiert werden. Hierzu werden die NCO-Gruppen mit solchen Verbindungen umgesetzt, die sich relativ leicht, zweckmäßigerweise bei erhöhter Temperatur, wieder zu NCO-Gruppen zurückspalten lassen. Die dabei freigesetzte reaktionsfähige Isocyanatgruppe kann dann in bekannter Weise, z.B. mit Hydroxyl- oder Aminogruppen, weiterreagieren. Geeignete Verkappungsmittel sind z.B. Phenole, Caprolactam, β-Dicarbonyl-Verbindungen, wie Acetessigester, Malonsäuredialkylester, Oxime, Triazole und bestimmte Alkohole. Modifizierte und/oder blockierte Polyisocyanatmischungen finden insbesondere Verwendung in hochwertigen Ein- und Zweikomponentensystemen für Lacke und Überzüge.

Bei Verwendung von organischen Polyisocyanaten mit Isocyanatgruppen unterschiedlicher Reaktivität, z.B. (cyclo)aliphatischen Diisocyanaten, wie 2-Ethyl-butandiisocyanat-1,4, 2-Methyl-pentandiisocyanat-1,5, 2,2,4-Trimethyl-hexandiisocyanat-1,6 (TMHDI) oder Isocyanatomethyl-3,5,5-trimethylcyclohexylisocyanat (IPDI), können durch Reaktion der reaktiveren Isocyanatgruppe modifizierte Polyisocyanatmischungen mit verminderter Reaktivität hergestellt werden, die sich durch längere Verarbeitungszeiten auszeichnen.

Es hat daher nicht an Versuchen gefehlt durch geeignete Modifizierung von (cyclo)aliphatischen Diisocyanaten mit Isocyanatgruppen unterschiedlicher Reaktivität ihre Lagerbeständigkeit, Verarbeitbarkeit und die mechanischen Eigenschaften daraus hergestellter Polyisocyanat-Polyadditionsprodukte zu verbessern.

Nach Angaben der EP-A-0 045 995 (US-A-4 476 054) können isocyanuratfreie Uretdione aus IPDI in Gegenwart eines Katalysators der Formel XₘP(NR₂)₃₋ₘ, in der bedeuten:
m 0, 1, 2, X Cl, OR, R und R gleiche oder verschiedene Alkyl-, Benzyl-, Phenylethyl-, Cyclohexyl- und Cyclopentylreste, hergestellt werden. Derartige uretdiongruppenhaltige IPDI können mit Diolen sowie gegebenenfalls Monoalkoholen oder Monoaminen gemäß EP-A-0 045 996 (US-A-4 483 798) zu uretdiongruppenhaltigen Polyadditionsprodukten umgesetzt werden, die nach ED-A-0 045 997 (US-A-4 430 474) verwendet werden können zur Herstellung von Einbrennlacken. Isocyanato-Uretdione und ein Verfahren zu ihrer Herstellung unter Verwendung von 2-Methyl-1,5-diisocyanatopentan und 2-Ethyl-1,4-diisocyanatobutan als geeignete Ausgangsstoffe werden ferner in der EP-A-0 099 976 (US-A-4 668 780) beschrieben. Die Uretdiongruppen enthaltenden Polyisocyanate sind Zwischenprodukte zur Herstellung von Kunststoffen, Lacken und Schaumstoffen.

Isocyanato-Isocyanurate und Verfahren zu ihrer Herstellung bzw. die Herstellung lagerstabiler Lösungen hiervon werden beschrieben in der EP-B-0 082 987 (US-A-4 469 867) und DE-B-2 325 826 (US-A-3 919 218). Nach Angaben der EP-A-0 082 987 werden zur Herstellung der Isocyanato-Isocyanurate 2-Methyl-1,5-diisocyanatopentan oder Mischungen aus 2-Methyl-1,5-diisocyanato-pentan und 2-Ethyl-1,4-diisocyanato-butan partiell trimerisiert. Gemäß DE-B-23 25 826 finden als (cyclo)aliphatische Diisocyanate IPDI oder 2,2,4- bzw. 2,4,4-TMHDI Verwendung.

Biuretgruppenhaltige Polyisocyanate aus 2-Methyl-1,5-diisocyanato-pentan oder 2-Ethyl-1,4-diisocyanato-butan werden in der EP-A-0 082 973 beschrieben.

Nachteilig an den beschriebenen modifizierten Polyisocyanaten ist deren Uneinheitlichkeit bezüglich ihrer chemischen Struktur und der Reaktivität der freien Isocyanatgruppen. Verursacht wird diese Uneinheitlichkeit durch die geringen Reaktivitätsunterschiede der zwei Isocyanatgruppen der als Ausgangsstoffe genannten (cyclo)aliphatischen Diisocyanate. Eine für bestimmte Anwendungen weitere unerwünschte mechanische Eigenschaft basiert auf der strukturbedingten Starrheit des IPDI-Moleküls.

Die Aufgabe der vorliegenen Erfindung bestand darin, modifizierte Polyisocyanatmischungen mit einer im Vergleich zu bekannten Produkten einheitlicheren Molekülzusammensetzung, verminderter Reaktivität und verlängerter Verarbeitungszeit herzustellen.

Diese Aufgabe konnte überraschenderweise gelöst werden durch eine Modifizierung von (cyclo)aliphatischen Diisocyanaten mit unterschiedlicher Reaktivität der Isocyanatgruppen, bedingt durch ihre spezielle chemische Struktur.

Gegenstand der Erfindung sind somit Urethan-, Allophanat-, Harnstoff-, Biuret-, Carbodiimid-, Uretdion- und/oder Isocyanuratgruppen enthaltende Polyisocyanatmischungen mit einem Isocyanatgehalt von 1 bis 34 Gew.-%, die hergestellt werden nach bekannten Verfahren aus (cyclo)aliphatischen Diisocyanaten, ausgewählt aus der Gruppe 2-Butyl-2-ethyl-pentan-diisocyanat-1,5, 2-(3-Isocyanatopropyl)-cyclohexylisocyanat-1, 1-(Isocyanatomethyl)-1-(3-isocyanatopropyl)-cyclohexan und Mischungen aus mindestens zwei der genannten (cyclo)aliphatischen Diisocyanate.

Überraschenderweise zeigte sich, daß die Reaktivitätsunterschiede der Isocyanatgruppen der erfindungsgemäß verwendbaren (cyclo)aliphatischen Diisocyanate, aufgrund der speziellen Substituenten und deren Einfluß auf die unmittelbare sterische Umgebung, wesentlich stärker ausgeprägt sind als bei den bekannten, vorgenannten (cyclo)aliphatischen, Diisocyanaten.

Beispielsweise zeichnet sich 2-(3-Isocyanatopropyl)-cyclohexylisocyanat durch eine sterisch ungehinderte primäre Isocyanatgruppe an einem Propylenrest und eine sekundäre Isocyanatgruppe am Cyclohexanring aus, während beim IPDI die primäre Isocyanatgruppe nur über eine Methylenbrücke (Neogruppierung) getrennt an den Cyclohexanring gebunden ist.

Aufgrund seiner flexiblen Struktur zeigt 2-Butyl-2-ethyl-pentan-diisocyanat-1,5 vorteilhafte Verarbeitungseigenschaften und ergibt Polyisocyanat-Polyadditionsprodukte mit hervorragenden mechanischen Eigenschaften. Die primäre Isocyanatgruppe am C₅-Atom ist über ein Propylenbrückenglied von den seitenständigen 2-Ethyl- und 2-Butylresten getrennt und daher reaktiver als die Isocyanatgruppe am C₁-Atom, die durch eine Neogruppierung sterisch gehindert und dadurch desaktiviert wird.

Da bei der Reaktion zur Modifizierung der erfindungsgemäß verwendbaren (cyclo)aliphatischen Diisocyanate, vorzugsweise die reaktivere primäre Isocyanatgruppe reagiert, besitzen die erfindungsgemäß modifizierten Polyisocyanatmischungen eine verminderte Reaktivität und hierdurch bedingt eine längere Verarbeitungszeit als mit analogen Gruppen modifizierte Polyisocyanatmischungen auf Basis von HDI, TMHDI oder IPDI.

Wie bereits dargelegt wurde, eignet sich zur Herstellung der erfindungsgemäß modifizierten Polyisocyanatmischungen 1-(Isocyanatomethyl)-1-(3-isocyanatopropyl)-cyclohexan. Als (cyclo)aliphatische Diisocyanate verwendet werden Jedoch vorzugsweise 2-(3-Isocyanatopropyl)cyclohexylisocyanat-1 (abgekürzt IPCI genannt) und insbesondere 2-Butyl-2-ethyl-pentan-diisocyanat-1,5 (abgekürzt BEPDI genannt). Die erfindungsgemäß verwendbaren (cyclo)aliphatischen Diisocyanate können nach beliebigen Verfahren, z.B. durch Phosgenierung der entsprechenden (cyclo)aliphatischen Diamine zu den entsprechenden Dicarbaminsäurechloriden und deren thermische Spaltung in die (cyclo)aliphatischen Diisocyanate und Chlorwasserstoff, hergestellt werden. Vorzugsweise Verwendung finden jedoch (cyclo)aliphatische Diisocyanate, insbesondere IPCI und BEPDI, die nach phosgenfreien Verfahren, insbesondere durch thermische Spaltung von (cyclo)aliphatischen Dicarbaminsäureestern in die (cyclo)aliphatischen Diisocyanate und Alkohole erhältlich sind. Diese thermische Spaltung kann beispielsweise bei Temperaturen von 150 bis 300°C, vorzugsweise 180 bis 250°C und Drücken von 0,001 bis 2 bar, vorzugsweise von 1 bis 200 mbar in Abwesenheit oder vorzugsweise Gegenwart von Katalysatoren in geeigneten Spaltreaktoren, wie z.B. Dünnschicht- oder vorzugsweise Heizkerzenverdampfern durchgeführt werden. Die bei der Spaltung entstehenden (cyclo)aliphatischen Diisocyanate und Alkohole können z.B. durch fraktionierte Kondensation oder vorzugsweise durch Rektifikation getrennt und die (cyclo)aliphatischen Diisocyanate beispielsweise durch Destillation zusätzlich gereinigt werden.

Die Herstellung der erfindungsgemäßen Urethan-, Allophanat-, Harnstoff-, Biuret-, Carbodiimid-, Uretdion- und/oder Isocyanuratgruppen enthaltenden Polyisocyanatmischungen auf Basis von 1-(Isocyanatomethyl)-1-(3-isocyanatopropyl)-cyclohexan, vorzugsweise IPCI und insbesondere BEPDI erfolgt nach an sich bekannten Methoden durch katalysatorfreie oder vorzugsweise katalytische Oligomerisierung und/oder Polykondensation der erfindungsgemäß verwendbaren (cyclo)aliphatischen Diisocyanate und/oder Polyaddition dieser Diisocyanate und/oder der erfindungsgemäß modifizierten Polyisocyanatmischungen an Verbindungen mit mindestens einem reaktiven Wasserstoffatom, vorzugsweise Wasser, mono- und/oder polyfunktionelle Alkohole und/oder mono- und/oder polyfunktionelle Amine.

Nach Erreichen des gewünschten Gehalts an Carbodiimid-, Uretdion- und/oder Isocyanuratgruppen in der Reaktionsmischung bzw. des gewünschten Umsetzungsgrades, der z.B. durch Bestimmung des Isocyanatgehalts der Reaktionsmischung ermittelt werden kann, kann die Reaktion durch Desaktivierung des Katalysators, beispielsweise durch Zugabe eines Katalysatorgifts oder durch thermische Zersetzung des Katalysators, gestoppt werden. Sofern monomerenfreie, modifizierte Polyisocyanatmischungen für bestimmte Anwendungsgebiete, wie z.B. Ein- oder Zweikomponenten-Polyurethanlacke, erforderlich sind, können die nicht umgesetzten monomeren Diisocyanate in geeigneter, an sich bekannter Weise, z.B. durch Destillation, beispielsweise mit Hilfe eines Dünnschichtverdampfers, abgetrennt werden.

Zur Herstellung von Urethan- und Allophanatgruppen aufweisenden Polyisocyanatmischungen können die erfindungsgemäß geeigneten (cyclo)aliphatischen Diisocyanaten mit Alkoholen, vorzugsweise Alkandiolen in den erforderlichen Mengen, vorteilhafterweise in Mengen von z.B. 0,005 bis 0,4, vorzugsweise 0,05 bis 0,15 Äquivalenten Hydroxylgruppe pro Äquivalent Isocyanatgruppe zur Reaktion gebracht werden. Harnstoff- und/oder Biuretgruppen enthaltende Polyisocyanatmischungen können durch Umsetzung der erfindungsgemäß verwendbaren (cyclo)aliphatischen Diisocyanate mit Wasser, Wasser abspaltenden Verbindungen, wie z.B. tertiär Butanol oder Kristallwasser aufweisenden Salzen, primären und sekundären Aminen, vorzugsweise aliphatischen und/oder aromatischen Diaminen, erhalten werden. Bei Verwendung eines tertiären Monoalkanols als Wasser abspaltende Verbindung ist das Molverhältnis von Diisocyanaten zu Alkohol üblicherweise mindestens 2,5:1, vorzugsweise mindestens 8:1.

Analogieverfahren zur Herstellung der erfindungsgemäß modifizierten Polyisocyanatmischungen werden in Literaturveröffentlichungen wie z.B. dem Kunststoff-Handbuch, Band 7, Polyurethane, Carl Hanser Verlag, 1. und 2. Auflage (1966 bzw. 1983), und insbesondere Patentpublikationen beschrieben.

Beschrieben werden beispielsweise Verfahren zur Herstellung von Urethangruppen enthaltenden Polyisocyanatmischungen in der DE-B-1 618 380 (US-A-3 644 457), DE-A-25 13 793 (GB-A-14 50 660) oder EP-A-10 850 (US-A-4 261 852), Allophanat- und Isocyanuratgruppen aufweisenden Polyisocyanatmischungen in der EP-A-0 566 037, Harnstoff- und/oder Biuretgruppen aufweisenden Polyisocyanatmischungen in der DE-B-1 101 394 (GB 876 503), DE-B-1 070 374, GB-A-889 050, DE-B-1 227 003 (US-A-3 284 479), DE-A-1 174 759 (US-A-3 392 183) und DE-A-1 543 178 (GB-A-1 044 932), Carbodiimidgruppen aufweisenden Polyisocyanatmischungen in der DE-A-1 130 594 (US-A-2 941 966), GB-A-1 083 410, US-A-2 941 983 und DE-B-22 48 751 (US-A- 4 076 945), Uretdiongruppen aufweisenden Polyisocyanatmischungen in der DE-A-1 643 170 (US 3 489 744) und DE-A-1 081 895 (GB-A-821 158) und insbesondere Isocyanuratgruppen aufweisenden Polyisocyanatmischungen in der DE-A-26 16 415, US-A-3 645 979, US 3 248 372 und GB-A-837 120.

Aus den erfindungsgemäß verwendbaren (cyclo)aliphatischen Diisocyanaten 1-(Isocyanatomethyl)-1-(3-isocyanatopropyl)-cyclohexan, vorzugsweise IPCI und insbesondere BEPDI werden insbesondere mit Isocyanuratgruppen modifizierte Polyisocyanatmischungen hergestellt, da diese zur Herstellung von hochwertigen Ein- oder Zweikomponenten-Polyurethan(PU)-klebstoffen oder insbesondere -lacken vorzüglich geeignet sind.

Zur Herstellung der erfindungsgemäßen Isocyanuratgruppen enthaltenden Polyisocyanatmischungen werden die erfindungsgemäß verwendbaren (cyclo)aliphatischen Diisocyanate üblicherweise, vorteilhafterweise unter einer Atmosphäre aus unter den Reaktionsbedingungen inerten Gasen, wie z.B. Stickstoff, in Gegenwart mindestens eines Trimerisierungskatalysators bei Temperaturen vorteilhafterweise von 30 bis 140°C, vorzugsweise von 70 bis 120°C partiell cyclisiert. Nach Erreichen des gewünschten Isocyanuratgehalts bzw. NCO-Gehalts, der vorteilhafterweise in einem NCO-Bereich von 1 bis 34 Gew.-%, vorzugsweise von 10 bis 34 Gew.-%, bezogen auf das Gewicht der Isocyanuratgruppen enthaltenden Reaktionsmischung, liegt und in Abhängigkeit von der gewählten Reaktionstemperatur im genannten Temperaturbereich üblicherweise in einem Zeitraum von 0,1 bis 12 Stunden, vorzugsweise von 2 bis 5 Stunden, erhalten wird, wird der Trimerisierungskatalysator durch Zugabe eines Desaktivators desaktiviert und dadurch die Isocyanuratbildung beendet. Die erhaltene Isocyanuratgruppen enthaltende Polyisocyanatmischung kann z.B. durch kontinuierliche oder stufenweise Zugabe von Verbindungen mit reaktiven Wasserstoffatomen, vorzugsweise Amino- oder insbesondere Hydroxylverbindungen zusätzlich modifiziert werden oder es können gegebenenfalls vor oder nach der zusätzlichen Modifizierung die monomeren organischen Diisocyanate, zweckmäßigerweise mit Hilfe eines Dünnschichtverdampfers unter vermindertem Druck, abgetrennt werden.

Zur partiellen Cyclisierung der erfindungsgemäß verwendbaren (cyclo)aliphatischen Diisocyanate können die üblichen Trimerisierungskatalysatoren, wie z.B. tertiäre Amine, Phosphine, Alkoholate, Metalloxide, Hydroxide, Carboxylate und metallorganische Verbindungen verwendet werden. Als Trimerisierungskatalysatoren vorzüglich bewährt haben sich ferner Tris-(N,N-dialkylaminoalkyl)-S-hexahydrotriazine, wie z.B. Tris-(N,N-dimethylaminopropyl)-S-hexahydrotriazin, und organische Salze von schwachen Säuren mit Tetraalkylammoniumgruppen oder Trialkylhydroxylalkylammoniumgruppen, wobei aufgrund ihrer einfachen Herstellungs- und Reinigungsweise z.B. N,N,N-Trimethyl-N-2-hydroxypropylammonium-p-tert.-butylbenzoat und N,N,N-Trimethyl-N-2-hydroxypropylammonium-2-ethylhexanoat bevorzugt sind. Überraschenderweise zeigte sich, daß auch die Art des Katalysators eine zusätzliche selektive Trimerisierung bewirken kann, wobei im Vergleich zu Tetraalkylammoniumcarboxylaten durch die Verwendung von Silylaminen, vorzugsweise von Bis(trimethylsilyl)amin, bevorzugt die reaktivere primäre Isocyanatgruppe aktiviert wird. Da durch diese selektive Trimerisierung eine zusätzliche Vereinheitlichung der Molekülstruktur und Reaktivität bewirkt wird, werden insbesondere Silylamine als Katalysatoren zur Herstellung der Isocyanuratgruppen enthaltenden Polyisocyanatmischungen verwendet. Die Trimerisierungskatalysatoren, die auch die Bildung von Uretdiongruppen und oligomeren Isocyanuratgruppen als Nebenprodukte bewirken können, werden üblicherweise in einer Menge von 0,001 bis 0,5 Gew.-%, vorzugsweise von 0,01 bis 0,05 Gew.-%, bezogen auf das Gewicht der organischen Diisocyanate, verwendet.

Wie bereits dargelegt wurde, werden nach Bildung der gewünschten Menge an Isocyanuratgruppen die Trimerisierungskatalysatoren üblicherweise desaktiviert. Als Desaktivatoren eignen sich im Falle der erstgenannten ionischen Katalysatoren beispielsweise anorganische oder organische Säuren, die entsprechenden Säurehalogenide und Alkylierungsmittel. Beispielhaft genannt seien Phosphorsäure, Monochloressigsäure, Dodecylbenzolsulfonsäure, Benzoylchlorid, Dimethylsulfat und vorzugsweise Dialkylphosphate, insbesondere Dibutylphosphat. Zur Desaktivierung der Silylamine als Katalysatoren werden vorzugsweise mono- und difunktionelle Alkohole, wie z.B. n-Butanol, n-Hexanol, 1,2-Propandiol und/oder 1,4-Butandiol verwendet. Die Desaktivierungsmittel können in Mengen von 1 bis 300 Mol-%, vorzugsweise 50 bis 150 Mol-%, bezogen auf die Mole an Trimerisierungskatalysator eingesetzt werden.

Obgleich die Isocyanurat- und gegebenenfalls Uretdiongruppen enthaltenden Polyisocyanate auch unter Mitverwendung von inerten Lösungs- oder Verdünnungsmitteln hergestellt werden könnten, finden diese beim erfindungsgemäßen Verfahren aus ökologischen und ökonomischen Gründen vorzugsweise keine Verwendung.

Die erfindungsgemäßen Isocyanuratgruppen enthaltenden Polyisocyanatmischungen enthalten zweckmäßigerweise zu mindestens 50 Gew.-%, vorzugsweise zu mindestens 60 Gew.-% und insbesondere zu mindestens 70 Gew.-%, bezogen auf die Isocyanuratpolyisocyanate, Polyisocyanate der Formel 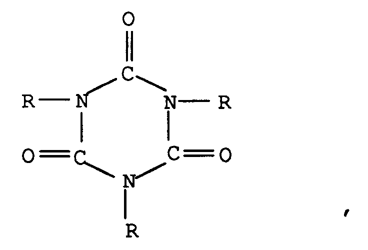
in der R einen Rest bedeutet, ausgewählt aus der Gruppe 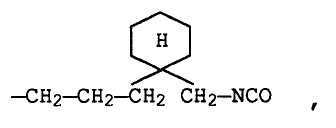
vorzugsweise 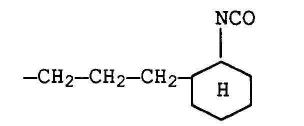
und insbesondere

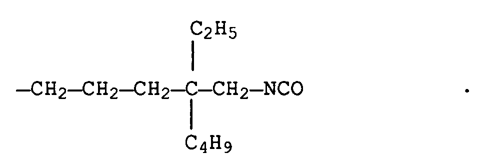

Die erfindungsgemäßen Urethan-, Allophanat-, Harnstoff-, Biuret-, Carbodiimid-, Uretdion- und/oder Isocyanuratgruppen enthaltenden Polyisocyanatmischungen besitzen einen Isocyanatgehalt im Bereich von 1 bis 34 Gew.-%, bezogen auf das Gesamtgewicht. Nach bevorzugten Ausführungsformen enthalten die Urethangruppen enthaltenden Polyisocyanatmischungen einen Isocyanatgehalt vorzugsweise von 1 bis 30 Gew.-%, insbesondere von 1,5 bis 26 Gew.-%, die Allophanatgruppen enthaltenden Polyisocyanatmischungen einen Isocyanatgehalt vorzugsweise von 1 bis 30 Gew.-%, insbesondere von 1,5 bis 26 Gew.-%, die Harnstoffgruppen enthaltenden Polyisocyanatmischungen einen Isocyanatgehalt vorzugsweise von 1 bis 30 Gew.-%, insbesondere von 3 bis 26 Gew.-%, die Biuretgruppen enthaltende Polyisocyanatmischungen einen Isocyanatgehalt vorzugsweise von 5 bis 34 Gew.-%, insbesondere von 5 bis 25 Gew.-%, die Carbodiimidgruppen enthaltende Polyisocyanatmischungen einen Iscyanatgehalt vorzugsweise von 2 bis 33 Gew.-%, insbesondere von 5 bis 30 Gew.-%, die Uretdiongruppen enthaltende Polyisocyanatmischungen einen Isocyanatgehalt vorzugsweise von 5 bis 33 Gew.-%, insbesondere von 10 bis 30 Gew.-% und die Isocyanuratgruppen enthaltende Polyisocyanatmischungen einen Isocyanatgehalt vorzugsweise von 10 bis 34 Gew.-%, insbesondere von 10 bis 30 Gew.-%, wobei die Gewichtsprozente jeweils bezogen sind auf das Gesamtgewicht der Polyisocyanatmischungen. Sofern die erfindungsgemäß modifizierten Polyisocyanatmischungen mit mehr als einer der erfindungswesentlichen Gruppen modifiziert wurden, z.B. einer Uretdion- und Isocyanuratgruppe, einer Carbodiimid- und Isocyanuratgruppe, einer Urethan- und Isocyanuratgruppe, einer Urethan- und Allophanatgruppe, einer Urethan- und mindestens einer Carbodiimidgruppe oder Harnstoff- und Biuretgruppe, so liegen die Isocyanatgehalte ebenfalls im vorgenannten für eine Gruppe genannten Isocyanatprozentbereich.

Die erfindungsgemäß modifizierten Polyisocyanatmischungen können in der erhaltenen Form Verwendung finden oder vor der Verarbeitung mit einem gegenüber Isocyanatgruppen inerten Lösungsmittel, z.B. zur Reduzierung der Viskosität und Verbesserung der Fließfähigkeit, verdünnt werden. Der Art des zweckmäßigerweise gegebenenfalls eingesetzten Lösungsmittels ist üblicherweise auch abhängig vom Verwendungszweck der erfindungsgemäß modifizierten Polyisocyanatmischungen. Als geeignete Lösungsmittel kommen beispielsweise die in der Lack- und Klebstoffindustrie üblichen, gegenüber Isocyanatgruppen inerten Lösungs- und Verdünnungsmittel in Betracht.

Die erfindungsgemäßen Urethan-, Allophanat-, Biuret-, Carbodiimid-, Uretdion- und/oder Isocyanuratgruppen enthaltenden Polyisocyanatmischungen eignen sich beispielsweise zur Herstellung von Polyurethan-Lacksystemen, wie z.B. Ein- oder Zweikomponenten-Lacken, Polyurethan-Beschichtungen, -Dispersionen, -Klebstoffen, wie z.B. Ein- oder Zweikomponenten-Klebstoffen, Polyurethan-Dichtungsmitteln, zelligen oder kompakten Polyurethan-Elastomeren, -Gießelastomeren und -Schaumstoffen, wie z.B. weichelastischen, halbharten oder harten Block- oder Formschaumstoffen sowie den entsprechenden Integralschaumstoffen.

### Beispiele

Die in den Beispielen genannte Viskosität wurde gemessen nach DIN 53019 mit einem Rotationsviskosimeter bei 23°C.

In den Beispielen und Vergleichsbeispielen wurden folgende Diisocyanate verwendet, die wie in der Erfindungsbeschreibung abgekürzt genannt wurden:
- HDI:: 1,6-Hexamethylen-diisocyanat(1,6-Diisocyanatohexan),
- TMHDI:: 2,2,4-Trimethyl-hexamethylen-diisocyanat-1,6,
- IPDI:: 3-Isocyanatomethyl-3,5,5-trimethylcyclohexylisocyanat (Isophoron-diisocyanat),
- IPCI:: 2-(3-Isocyanatopropyl)-cyclohexylisocyanat-1 und
- BEPDI:: 2-Butyl-2-ethyl-pentan-diisocyanat-1,5.

### Beispiel 1

### Herstellung einer Biuretgruppen enthaltenden Polyisocyanatmischung auf Basis von IPCI.

In einem Mehrhalskolben, ausgestattet mit Rührer, Rückflußkühler und Septum, wurden 500 Gew.-Teile IPCI mit einem NCO-Gehalt von 40,3 Gew.-% und 15 Gew.-Teile tert.-Butanol unter Rühren gemischt und die Reaktionsmischung auf 160°C erwärmt.

In die Reaktionsmischung ließ man 0,5 Gew.-Teile para-Toluolsulfonsäure, gelöst in 2,8 Gew.-Teilen tert.-Butanol, in einem Zeitraum von 10 Minuten eintropfen. Es setzte eine heftige Entwicklung von Kohlendioxid und Isobuten ein, die nach ungefähr 30 Minuten beendet war. Zur Vervollständigung der Umsetzung wurde die Reaktionsmischung noch 60 Minuten bei 160°C gerührt, danach auf Raumtemperatur abkühlen gelassen und bei Raumtemperatur unter vermindertem Druck (ca. 20 mbar) 30 minutenlang entgast. Aus der erhaltenen Polyisocyanatmischung, die einen Isocyanatgehalt von 32,9 Gew.-% aufwies, wurde mit Hilfe eines Dünnschichtverdampfers das nicht umgesetzte Monomere IPCI abdestilliert.

Man erhielt eine zähe, gelb gefärbte Biuret- und Uretdiongruppen enthaltende Polyisocyanatmischung mit einem Isocyanatgehalt von 19,7 Gew.-% und einer Viskosität bei 23°C von größer 10 000 mPas.

### Beispiel 2

### Herstellung einer Biuretgruppen enthaltenden Polyisocyanatmischung auf Basis BEPDI.

Man verfuhr analog den Angaben von Beispiel 1, verwendete jedoch als Ausgangsstoffe 700 Gew.-Teile BEPDI mit einem Isocyanatgehalt von 34,8 Gew.-%, 24 Gew.-Teile tert.-Butanol und 0,7 Gew.-Teile para-Toluolsulfonsäure, gelöst in 2,0 Gew.-Teilen tert.-Butanol.

Aus der erhaltenen Polyisocyanatmischung, die einen Isocyanatgehalt von 20,3 Gew.-% aufwies, wurde mit Hilfe eines Dünnschichtverdampfers das nicht umgesetzte Monomere BEPDI abdestilliert.

Man erhielt eine zähe, gelbgefärbte Biuretgruppen enthaltende Polyisocyanatmischung mit einem Isocyanatgehalt von 16,1 Gew.% und einer Viskosität bei 23°C von größer 10 000 mPas.

### Beispiel 3

### Herstellung einer Urethan- und Allophanatgruppen enthaltenden Polyisocyanatmischung auf Basis BEPDI.

In einem Mehrhalskolben, ausgestattet mit Rührer, Gasein- und ableitungsvorrichtung, Rückflußkühler und Septum, wurden unter einer Stickstoffatmosphäre 500 Gew.-Teile BEPDI mit einem Isocyanatgehalt von 34,8 Gew.-%, 31,1 Gew.-Teile n-Butanol und 0,27 Gew.-Teile Kupfer(II)acetylacetonat gemischt, die Reaktionsmischung auf 80°C erwärmt und bei dieser Temperatur 6 Stunden lang zur Reaktion gebracht. Zu der erhaltenen Reaktionsmischung, die einen Isocyanatgehalt von 23,6 Gew.-% besaß, fügte man 0,44 Gew.-Teile Dibutylphosphat und destillierte anschließend das nicht umgesetzte BEPDI mittels eines Dünnschichtverdampfers unter vermindertem Druck (ca. 3 mbar) ab.

Man erhielt eine ölige, Urethan- und Allophanatgruppen enthaltende Polyisocyanatmischung mit einem Isocyanatgehalt von 15,2 Gew.-% und einer Viskosität bei 23°C von 6140 mPas.

### Beispiel 4

### Herstellung einer Allophanat- und Isocyanuratgruppen enthaltenden Polyisocyanatmischung.

In einem wie in Beispiel 3 beschrieben ausgestatteten Mehrhalskolben wurde eine Mischung aus 500 Gew.-Teilen BEPDI mit einem Isocyanatgehalt von 34,8 Gew.-% und 13,8 Gew.-Teilen 2-Ethylhexanol unter einer Stickstoffatmosphäre auf 100°C erwärmt und bei dieser Temperatur eine Stunde zur Reaktion gebracht. Die Reaktionsmischung, die man auf 80°C abkühlen ließ, besaß danach einen Isocyanatgehalt von 32,9 Gew.-%.

Bei dieser Temperatur fügte man 0,2 Gew.-%, bezogen auf das Gewicht des eingesetzten BEPDI, Trimethyl-2-hydroxypropylammonium-2-ethylhexanoat als Trimerisierungskatalysator zur Reaktionsmischung, rührte diese bei 80°C 4 Stunden lang und desaktivierte den Trimerisierungskatalysator durch Zugabe von 0,2 Gew.-% Dibutylphosphat, bezogen auf das Gewicht des eingesetzten BEPDI. Aus der erhaltenen Reaktionsmischung, die einen Isocyanatgehalt von 27 Gew.-% besaß, wurde das monomere BEPDI mittels eines Dünnschichtverdampfers abdestilliert.

Man erhielt eine klare, gelbliche Allophanat- und Isocyanuratgruppen enthaltende Polyisocyanatmischung mit einem Isocyanatgehalt von 14,3 Gew.-% und einer Viskosität bei 23°C von größer 10 000 mPas.

### Beispiel 5

### Herstellung einer Isocyanurat- und Urethangruppen enthaltenden Polyisocyanatmischung

In einem wie in Beispiel 3 beschrieben ausgestatteten Mehrhalskolben fügte man zu 500 Gew.-Teilen BEPDI 0,2 Gew.-%, bezogen auf das BEPDI, Trimethyl-2-hydroxypropylammonium-2-ethyl-hexanoat, erwärmte die Reaktionsmischung unter einer Stickstoffatmosphäre auf 80°C und cyclisiert das BEPDI bei dieser Temperatur 4 Stunden lang. Danach wurde der Trimerisierungskatalysator durch Zugabe von 0,2 Gew.-% Dibutylphosphat, bezogen auf das BEPDI-Gewicht, desaktiviert und die Reaktionsmischung, die einen Isocyanatgehalt von 29,4 Gew.-% aufwies, mit 13,8 Gew.-Teilen 2-Ethylhexanol zur Reaktion gebracht. Nach einer Nachreaktionszeit von 2 Stunden bei 80°C besaß die Reaktionsmischung einen Isocyanatgehalt von 27,2 Gew.-%. Nach dem Abdestillieren des nicht umgesetzten monomeren BEPDI mittels Dünnschichtdestillation unter vermindertem Druck erhielt man eine klare gelbliche Isocyanurat- und Urethangruppen enthaltende Polyisocyanatmischung mit einem Isocyanatgehalt von 13,5 Gew.-% und einer Viskosität bei 23°C von größer 10 000 mPas.

### Beispiel 6

### Herstellung einer Isocyanurat- und Uretdiongruppen enthaltenden Polyisocyanatmischung.

In einem Mehrhalskolben, ausgestattet mit Rührer, Rückflußkühler und Gasein- und -ableitungsvorrichtung, wurde unter einer Stickstoffatmosphäre eine Reaktionsmischung aus 500 Gew.-Teilen IPCI mit einem Isocyanatgehalt von 40,3 Gew.-% und 0,1 Gew.-%, bezogen auf das IPCI-Gewicht, Trimethyl-2-hydroxypropylammonium-2-ethylhexanoat auf 80°C erwärmt und bei dieser Temperatur unter Rühren 3 Stunden lang cyclisiert. Der Trimerisierungskatalysator wurde durch Zugabe von 0,1 Gew.-% Dibutylphosphat, bezogen auf das IPCI-Gewicht, desaktiviert. Aus der Reaktionsmischung, die einen Isocyanatgehalt von 33 Gew.-% besaß, wurde das nicht umgesetzte IPCI mittels Dünnschichtdestillation unter vermindertem Druck abdestilliert.

Man erhielt eine klare, gelbliche Isocyanurat- und Uretdiongruppen enhaltende Polyisocyanatmischung mit einem Isocyanatgehalt von 19,3 Gew.-% und einer Viskosität bei 23°C von größer 10 000 mPas.

Die Isocyanurat- und Uretdiongruppen enthaltende Polyisocyanatmischung wurde zur leichteren Weiterverarbeitung mit n-Butylacetat zu einer 90 gew.-%igen Lösung verdünnt, die einen Isocyanatgehalt von 17,3 Gew.-% besaß und eine Viskosität bei 25°C von 480 mPas aufwies.

### Beispiel 7

### Herstellung einer Isocyanurat- und Uretdiongruppen enthaltenden Polyisocyanatmischung.

In einem wie in Beispiel 6 beschrieben ausgestatteten Mehrhalskolben wurde unter einer Stickstoffatmosphäre eine Reaktionsmischung aus 500 Gew.-Teilen BEPDI mit einem Isocyanatgehalt von 34,8 Gew.-% und 0,2 Gew.-%, bezogen auf das BEPDI-Gewicht, Trimethyl-2-hydroxypropylammonium-2-ethylhexanoat auf 80°C erwärmt und bei dieser Temperatur unter Rühren 4 Stunden lang cyclisiert. Der Trimerisierungskatalysator wurde durch Zugabe von 0,2 Gew.-% Dibutylphosphat, bezogen auf das BEPDI-Gewicht, desaktiviert. Aus der Reaktionsmischung wurde das nicht umgesetzte BEPDI mittels Dünnschichtdestillation unter vermindertem Druck abdestilliert.

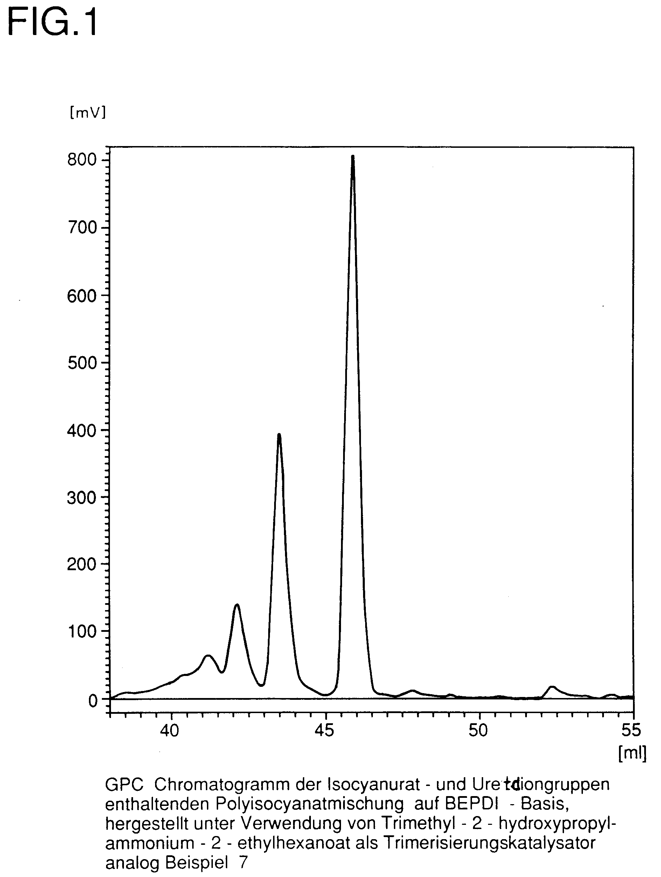

Man erhielt eine klare, gelbliche, amorphe Isocyanurat- und Uretdiongruppen enhaltende Polyisocyanatmischung mit einem Isocyanatgehalt von 15,5 Gew.-%, dessen GPC-Chromatogramm Abbildung 1 zeigt.

Die Isocyanurat- und Uretdiongruppen enthaltende Polyisocyanatmischung wurde zur leichteren Weiterverarbeitung mit n-Butylacetat zu einer 90 gew.-%igen Lösung verdünnt, die einen Isocyanatgehalt von 14,3 Gew.-% besaß und eine Viskosität bei 25°C von 440 mPas aufwies.

### Beispiel 8

### Herstellung einer Isocyanurat- und Uretdiongruppen enthaltenden Polyisocyanatmischung.

In einem wie in Beispiel 6 beschrieben ausgestatteten Mehrhalskolben wurde unter einer Stickstoffatmosphäre eine Reaktionsmischung aus 500 Gew.-Teilen BEPDI mit einem Isocyanatgehalt von 34,8 Gew.-% und 2 Gew.-% Bis(trimethylsilyl)amin (Hexamethyldisilazan) auf 110°C erwärmt und bei dieser Temperatur 12 Stunden lang unter Rühren cyclisiert. Der Reaktionsmischung wurden danach 0,2 Gew.-%, bezogen auf das BEPDI-Gewicht, n-Butanol hinzugefügt und die Reaktionsmischung nochmals 30 Minuten bei 110°C gerührt. Aus der erhaltenen Reaktionsmischung, die einen Isocyanatgehalt von 28,9 Gew.-% besaß, wurde das nicht umgesetzte BEPDI mittels Dünnschichtdestillation unter vermindertem Druck abdestilliert.

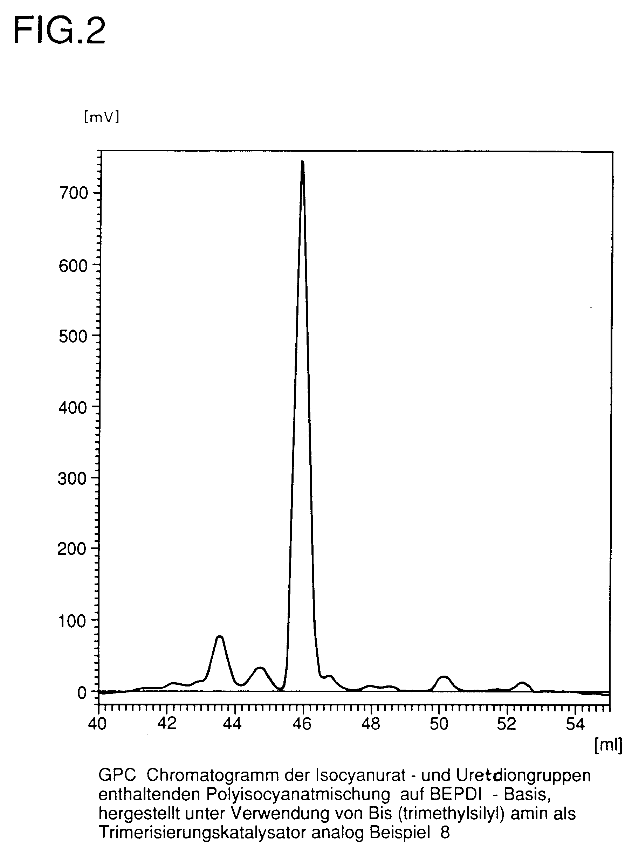

Man erhielt eine klare, gelbliche, amorphe Isocyanurat- und Uretdiongruppen enthaltende Polyisocyanatmischung mit einer deutlich engeren Molekulargewichtsverteilung als bei Beispiel 7 und mit einem Isocyanatgehalt von 16,9 Gew.-%, dessen GPC-Chromatogramm Abbildung 2 zeigt.

Die über ¹H-NMR-Spektroskopie bestimmte Selektivität bzw. Reaktivität der primären Isocyanatgruppe gegenüber der Isocyanatgruppe mit einer Neokonfiguration beträgt bei BEPDI und der Verwendung als Trimerisierungskatalysator von Trimethyl-2-hydroxypropylammonium-2-ethylhexanoat ungefähr 3:1 (Beispiel 7) und von Bis(trimethylsilyl)amin ungefähr 8:1 (Beispiel 8).

### Vergleichsbeispiele

### Herstellung von Isocyanurat- und Uretdiongruppen enthaltenden Polyisocyanatmischungen auf Basis von HDI, TMHDI und IPDI.

### Vergleichsbeispiel I

In einem wie in Beispiel 6 beschrieben ausgestatteten Mehrhalskolben wurden unter einer Stickstoffatmosphäre bei 80°C 500 Gew.-Teile HDI mit einem Isocyanatgehalt von 50 Gew.-% und 2 Gew.-%, bezogen auf das HDI-Gewicht, Bis(trimethylsilyl)amin (Hexamethyldisilazan) gemischt, danach die Reaktionsmischung auf 110°C erhitzt und bei dieser Temperatur 5 Stunden lang unter Rühren cyclisiert. Der Reaktionsmischung wurden danach 1,5 Gew.-%, bezogen auf das HDI-Gewicht, n-Butanol hinzugefügt und die Reaktionsmischung nochmals 30 Minuten bei 110°C gerührt. Aus der erhaltenen Reaktionsmischung, die einen Isocyanatgehalt von 41,2 Gew.-% besaß, wurde das nicht umgesetzte HDI mittels Dünnschichtdestillation unter vermindertem Druck (ca. 20 mbar) abdestilliert.

Man erhielt eine klare, viskose Isocyanurat- und Uretdiongruppen enthaltende Polyisocyanatmischung mit einem Isocyanatgehalt von 22,9 Gew.-% und einer Viskosität bei 23°C von 1800 mPas.

### Vergleichsbeispiel II

In einem wie in Beispiel 6 beschrieben ausgestatteten Mehrhalskolben wurden unter einer Stickstoffatmosphäre bei 80°C 500 Gew.-Teile TMHDI mit einem Isocyanatgehalt von 40 Gew.-% und 2 Gew.-%, bezogen auf das TMHDI-Gewicht, Bis(trimethylsilyl)amin (Hexamethyldisilazan) gemischt, danach die Reaktionsmischung auf 110°C erhitzt und bei dieser Temperatur 12 Stunden lang unter Rühren cyclisiert. Der Reaktionsmischung wurden danach 1,0 Gew.-%, bezogen auf das TMHDI-Gewicht, n-Butanol hinzugefügt und die Reaktionsmischung nochmals 30 Minuten bei 110°C gerührt. Aus der erhaltenen Reaktionsmischung, die einen Isocyanatgehalt von 28,9 Gew.-% besaß, wurde das nicht umgesetzte TMHDI mittels Dünnschichtdestillation unter vermindertem Druck (ca. 20 mbar) abdestilliert.

Man erhielt eine gelbliche, klare, viskose Isocyanurat- und Uretdiongruppen enthaltende Polyisocyanatmischung mit einem Isocyanatgehalt von 19 Gew.-% und einer Viskosität bei 23°C von 7600 mPas.

### Vergleichsbeispiel III

In einem wie in Beispiel 6 beschrieben ausgestatteten Mehrhalskolben wurden unter einer Stickstoffatmosphäre bei 80°C 500 Gew.-Teile IPDI mit einem Isocyanatgehalt von 37,8 Gew.-% und 2 Gew.-%, bezogen auf das IPDI-Gewicht, Bis(trimethylsilyl)amin (Hexamethyldisilazan) gemischt, danach die Reaktionsmischung auf 110°C erhitzt und bei dieser Temperatur 7 Stunden lang unter Rühren cyclisiert. Der Reaktionsmischung wurden danach 1,0 Gew.-%, bezogen auf das IPDI-Gewicht, n-Butanol hinzugefügt und die Reaktionsmischung nochmals 30 Minuten bei 110°C gerührt. Aus der erhaltenen Reaktionsmischung, die einen Isocyanatgehalt von 29,2 Gew.-% besaß, wurde das nicht umgesetzte IPDI mittels Dünnschichtdestillation unter vermindertem Druck (ca. 20 mbar) abdestilliert.

Man erhielt eine gelbliche, klare, viskose Isocyanurat- und Uretdiongruppen enthaltende Polyisocyanatmischung mit einem Isocyanatgehalt von 17,3 Gew.-%, die beim Stehenlassen bei Raumtemperatur kristallisierte und dann einen Schmelzbereich von 90 bis 100°C besaß.

Die Verteilung der cyclischen und linearen Oligomeren der bei einem Diisocyanatumsatz von ungefähr 30 % nach Beispiel 8 und den Vergleichsbeispielen I bis III hergestellten Isocyanurat- und Uretdiongruppen enthaltenden Polyisocyanatmischungen, bestimmt über die Flächenprozente der Gel-Permeations-Chromatographie (GPC) sind in der nachfolgenden Tabelle zusammengefaßt.

In der Tabelle bedeuten:
- Isocyanurat (1):: Isocyanuratgruppen enthaltendes Triisocyanat, erhalten aus 3 Mol Diisocyanat
- Isocyanurat (2):: Diisocyanuratgruppen enthaltendes Tetraisocyanat, erhalten aus 5 Mol Diisocyanat
- Isocyanurat (3):: Oligomere Isocyanuratgruppen enthaltende Polyisocyanate, erhalten aus mindestens 6 Mol Diisocyanat.

**Tabelle**

| Beispiel | 8 | | | |
|---|---|---|---|---|
| Vergleichsbeispiel | | I | II | III |
| Diisocyanat | BEPDI | HDI | TMHDI | IPDI |
| Isocyanurat (1) [Flächenprozent] | 80 | 53 | 50 | 50 |
| Isocyanurat (2) [Flächenprozent] | 10 | 19 | 18 | 10 |
| Isocyanurat (3) [Flächenprozent] | 5 | 18 | 18 | 5 |
| Nebenprodukte, u.a. Uretdiongruppen [Flächenprozent] | 5 | 10 | 14 | 35 |

Die hohe Selektivität des erfindungsgemäß verwendbaren BEPDI zeigte sich durch den hohen Gehalt an Isocyanuratgruppen enthaltendem Triisocyanat (Isocyanurat (1)) in Verbindung mit dem geringen Gehalt an mindestens 2 Isocyanuratringen enthaltenden Polyisocyanaten und Nebenprodukten (Beispiel 8) aus.

## Patentansprüche

1. Urethan-, Allophanat-, Harnstoff-, Biuret-, Carbodiimid-, Uretdion- und/oder Isocyanuratgruppen enthaltende Polyisocyanatmischungen mit einem Isocyanatgehalt von 1 bis 34 Gew.-%, hergestellt nach bekannten Verfahren aus Diisocyanaten, ausgewählt aus der Gruppe 2-Butyl-2-ethyl-pentan-diisocyanat-1,5, 2-(3-Isocyanatopropyl)-cyclohexylisocyanat-1, 1-(Isocyanatomethyl)-1-(3-isocyanatopropyl)-cyclohexan und Mischungen der genannten Diisocyanate.

2. Urethan-, Allophanat-, Harnstoff-, Biuret-, Carbodiimid-, Uretdion- und/oder Isocyanuratgruppen enthaltende Polyisocyanatmischungen mit einem Isocyanatgehalt von 1 bis 34 Gew.-%, erhältlich aus 2-Butyl-2-ethyl-pentan-diisocyanat-1,5 nach bekannten Verfahren.

3. Isocyanuratgruppen enthaltende Polyisocyanatmischungen mit einem Isocyanatgehalt von 10 bis 34 Gew.-%, enthaltend zu mindestens 50 Gew.-%, bezogen auf die Isocyanuratpolyisocyanate, Polyisocyanate der Formel 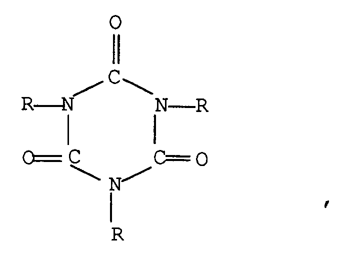 in der R einen Rest bedeutet, ausgewählt aus der Gruppe 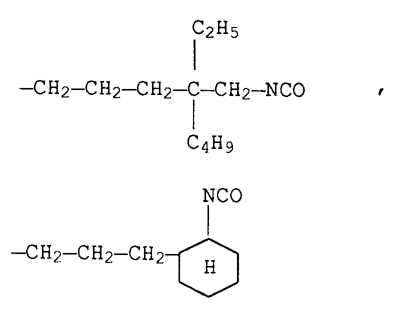 und
Zeichn.

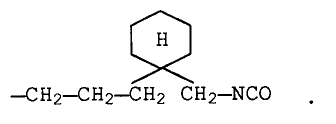

4. Isocyanuratgruppen enthaltende Polyisocyanatmischungen nach Anspruch 3, dadurch gekennzeichnet, daß der Rest R aus 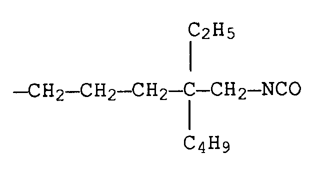 besteht.

5. Urethangruppen enthaltende Polyisocyanatmischungen mit einem Isocyanatgehalt von 1 bis 34 Gew.-%, erhältlich nach bekannten Verfahren aus Diisocyanaten, ausgewählt aus der Gruppe 2-Butyl-2-ethyl-pentan-diisocyanat-1,5, 2-(3-Isocyanatopropyl)-cyclohexylisocyanat-1, 1-(Isocyanatomethyl)-1-(3-isocyanatopropyl)-cyclohexan und Mischungen der genannten Diisocyanate.

6. Biuretgruppen enthaltende Polyisocyanatmischungen mit einem Isocyanatgehalt von 5 bis 34 Gew.-%, erhältlich nach bekannten Verfahren aus Diisocyanaten, ausgewählt aus der Gruppe 2-Butyl-2-ethyl-pentan-diisocyanat-1,5, 2-(3-Isocyanatopropyl)-cyclohexylisocyanat-1, 1-(Isocyanatomethyl)-1-(3-isocyanatopropyl)-cyclohexan und Mischungen der genannten Diisocyanate.

7. Allophanat- und/oder Urethangruppen enthaltende Isocyanurat-Polyisocyanatmischungen mit einem Isocyanatgehalt von 1 bis 34 Gew.-%, erhältlich nach bekannten Verfahren aus Diisocyanaten, ausgewählt aus der Gruppe 2-Butyl-2-ethyl-pentan-diisocyanat-1,5, 2-(3-Isocyanatopropyl)-cyclohexylisocyanat-1, 1-(Isocyanatomethyl)-1- (3-isocyanatopropyl)-cyclohexan und Mischungen der genannten Diisocyanate.

8. Verfahren zur Herstellung von Isocyanuratgruppen enthaltenden Polyisocyanatmischungen mit einem Isocyanatgehalt von 1 bis 34 Gew.-%, dadurch gekennzeichnet, daß man mindestens ein Diisocyanat, ausgewählt aus der Gruppe 2-Butyl-2-ethyl-pentan-diisocyanat-1,5, 2-(3-Isocyanatopropyl)-cyclohexylisocyanat-1 und 1-(Isocyanatomethyl)-1-(3-isocyanatopropyl)-cyclohexan in Gegenwart von Bis-(trimethylsilyl)amin trimerisiert.

9. Verfahren nach Anspruch 8, dadurch gekennzeichnet, daß man die Trimerisierung bei 30 bis 140°C durchführt.

10. Verwendung der Urethan-, Allophanat-, Biuret-, Carbodiimid-, Uretdion- und/oder Isocyanuratgruppen enthaltenden Polyisocyanatmischungen nach einem der Ansprüche 1 bis 7 zur Herstellung von Polyurethan-Lacksystemen, -Beschichtungen, -Dispersionen, -Klebstoffen, -Dichtungsmitteln, zelligen oder kompakten Polyurethan-Elastomeren und -Schaumstoffen.
